# EUROPEAN PATENT APPLICATION

(11) **EP 3 346 808 A1**
(43) Date of publication of application: **11.07.2018**
(21) Application number: 17150571.2
(22) Date of filing: 06.01.2017
(51) Int. Cl.: H05H 1/34

(54) **PLANAR DEVICE AND METHOD FOR GENERATING A PLASMA OR REACTIVE SPECIES**

(71) Applicant: INP Greifswald Leibniz-Institut für Plasmaforschung und Technologie e. V., 17489 Greifswald (DE)
(72) Inventor: SCHMIDT-BLEKER, Ansgar, 17489 Greifswald (DE); WINTER, Jörn, 17489 Greifswald (DE); WELTMANN, Klaus-Dieter, 18609 Binz (DE)
(74) Representative: Schulz Junghans Patentanwälte PartGmbB

(57) **Abstract**

The invention relates to a device (100) for generating a plasma (180) and/ or reactive species comprising at least one discharge chamber (400) for generating a plasma (180) and at least one nozzle (130) for generating a negative pressure, particularly between 10 mbar and 900 mbar, in the at least one discharge chamber (400), or at least one discharge channel (500), wherein the at least one discharge channel (500) is adapted to generate a plasma (180) and a negative pressure, particularly between 10 mbar and 900 mbar, wherein the at least one discharge chamber (400) and the at least one nozzle (130) or the at least one discharge channel (500) extend between a first plane and a second plane, wherein the first plane and the second plane are parallel.

The invention further relates to a method for generating a plasma (180) and a method for generating a reactive species.

## Description

The invention relates to a planar device for generating a plasma and/ or reactive species, a method for generating a plasma, and a method for generating a reactive species, particularly for sterilizing and/ or disinfecting a sample.

Generating reactive species, particularly at atmospheric or near-atmospheric pressure, is important for biomedical applications such as plasma-mediated decontamination of surfaces and plasma medical therapy.

For these applications, the plasma itself is usually also generated at atmospheric pressure, which is disadvantageous because relatively high voltages are required to ignite the plasma, which induces high costs for high voltage generation and electromagnetic shielding. Furthermore, ozone is formed in relatively high concentrations in plasma using a process gas comprising oxygen due to the higher probability for ternary collisions of molecules at atmospheric or near-atmospheric pressure than at reduced pressure. Ozone is a very effective disinfectant. However, since ozone is suspected to be carcinogenic and has a distinctive unpleasant smell, it is often desirable to keep the ozone concentration at a minimum and/or prevent its unintended inhalation.

Therefore, devices and methods for generating a plasma at reduced pressure have been developed. For example, the document WO 2008/138504 A1 discloses a device for plasma generation, in which a negative pressure is generated by means of a pressure gas flow through a cylindrical Laval nozzle constituting a part of the discharge chamber.

Due to its cylindrical design, this device is relatively heavy and bulky. Furthermore, the nozzle is preferably made of one piece and hence cannot provide two electrodes in the nozzle.

Thus, the problem to be solved by the present invention is to provide a device and a method for generating a plasma at reduced pressure, which is improved with respect to the disadvantages of the prior art, particularly a device which is reduced in size and weight compared to the prior art.

This problem is solved by the subject matter of the independent device claim 1, the independent method claims 13 and 14, and the independent use claim 15. Favorable embodiments of the invention are claimed by the dependent device claims 2 to 12. The invention is described in detail hereafter.

A first aspect of the invention relates to a device for generating a plasma and/ or reactive species comprising
- according to a first alternative at least one discharge chamber for generating a plasma and at least one nozzle for generating a negative pressure, particularly between 10 mbar and 900 mbar, in the at least one discharge chamber, or
- according to a second alternative at least one discharge channel, wherein the at least one discharge channel is adapted to generate a plasma and a negative pressure, particularly between 10 mbar and 900 mbar, in the discharge channel, wherein
- according to the first alternative, the at least one discharge chamber and the at least one nozzle extend between a first plane and a second plane, wherein the first plane and the second plane are parallel, or
- according to the second alternative, the at least one discharge channel extends between a first plane and a second plane, wherein the first plane and the second plane are parallel.

In other words, the at least one discharge chamber, the at least one nozzle, and/ or the at least one discharge channel comprises a flat, planar shape.

In the context of the present specification, the term "extends between a first plane and a second plane" means that a cavity forming the at least one discharge chamber, the at least one nozzle, and/ or the at least one discharge channel stretches between essentially planar surfaces. In particular, the respective cavity may have a square-shaped, essentially square-shaped, rectangular-shaped or essentially rectangular-shaped cross-section. That is, in particular, the corners of an essentially square-shaped or rectangular-shaped cross-section may be rounded. Alternatively, in particular, the first and/ or the second plane may not be completely planar, but comprise a slight curvature, wherein the radius of curvature is at least 4 cm.

In the context of the present specification, the term nozzle designates a cavity extending along a longitudinal axis, wherein the cross-sectional extension (or diameter in case of a circular-shaped cross-section) of the cavity varies along the longitudinal axis. Due to the varying extension, a negative pressure can be generated by the nozzle. For example, a Venturi nozzle comprises a central constriction of minimal extension, where the static pressure is lowest, and the dynamic pressure is highest.

In order to generate the negative pressure in the at least one discharge chamber, the at least one nozzle is in flow connection or can be brought into flow connection with the at least one discharge chamber, for example by means of at least one suction channel. Therein, a suction gas flow between the at least one discharge chamber and the at least one nozzle, particularly via the suction channel, is generated.

Generating the plasma at reduced pressure relative to the atmospheric pressure advantageously reduces the necessary voltage to ignite the plasma and results in less formation of ozone when process gases comprising oxygen are used. In addition, a reduced pressure facilitates formation of nitrogen oxides, particularly nitrogen monoxide, which is advantageous for some applications of the device, as specified below.

In the context of the present specification, the term discharge channel refers to a nozzle-shaped channel, which is designed as a discharge chamber. That is, the discharge channel is adapted such that a plasma discharge may be generated in the discharge channel, for example by electrodes incorporated in the walls of the discharge channel or by electrodes positioned outside of the discharge channel. Due to its nozzle-like shape, the discharge channel is also adapted to generate a reduced (or negative pressure) in the discharge channel. In particular, the at least one discharge channel is adapted to generate a gliding arc plasma.

In particular, the device may comprise two or more nozzles or discharge channels, which are connected in parallel or in series. Thereby, particularly a higher flow rate of the suction gas flow can be achieved compared to a single nozzle or discharge channel.

The surface normal of the first and second plane is also termed first longitudinal axis.

The device is adapted to generate a plasma and/ or a reactive species.

In the context of the present specification, the term reactive species refers to atoms, molecules, or ions, particularly radicals, which after their generation undergo chemical reactions with their own species or other species, and therefore cannot be stored for more than one hour.

In particular, the reactive species may be generated from the plasma generated by the device, or the reactive species may be subsequently generated from other species produced by means of the plasma.

In particular, a product gas and/ or product gas flow generated by the device may comprise the reactive species.

The flat shape of the device is advantageous for many applications. In particular the device can be incorporated in a handheld unit. Furthermore, the device can be built at a reduced size and weight compared to similar devices of the prior art. This is especially advantageous if short-lived and/ or unstable reactive species are generated by the plasma produced by the device, because a small and light plasma device can be transported to the site of application, and the reactive species can be generated directly at the site of application. For example, reactive species may be generated by the device according to the invention on site for surface for disinfection or decontamination.

Furthermore, due to its flat, planar design, the device is especially easy to manufacture. In particular, the at least one discharge chamber, the at least one nozzle and/ or the at least one discharge channel may be formed by recesses or apertures in a main body of the device, for example by cutting, milling or ablation techniques. Moreover, the device can also be easily assembled from modular components, for example half shells and/ or connecting pieces, wherein particularly the at least one discharge chamber, the at least one nozzle and/ or the at least one discharge channel may be formed by separate or combined recesses or apertures in the modular components.

In particular, the at least one discharge chamber comprises a maximum height extending along the first longitudinal axis and a maximum extension extending perpendicular to the first longitudinal axis, wherein the ratio between the maximum height and the maximum extension is 1 to 5 or less, more particularly 1 to 10 or less, most particularly 1 to 20 or less.

In particular, the at least one nozzle comprises a maximum height extending along the first longitudinal axis and a maximum extension extending perpendicular to the first longitudinal axis, wherein the ratio between the maximum height and the maximum extension is 1 to 5 or less, more particularly 1 to 10 or less, most particularly 1 to 20 or less.

In particular, the at least one discharge channel comprises a maximum height extending along the first longitudinal axis and a maximum extension extending perpendicular to the first longitudinal axis, wherein the ratio between the maximum height and the maximum extension is 1 to 5 or less, more particularly 1 to 10 or less, most particularly 1 to 20 or less.

In certain embodiments, the at least one discharge chamber or the at least one discharge channel is sealed from the environment of the device.

The at least one discharge chamber may have any geometric shape, for example a circular, elliptic, or angular shape, or a combination of these.

In certain embodiments, the plasma is a dielectric barrier discharge (DBD), a micro hollow cathode discharge (MHCD), a corona discharge, a microwave plasma, a capacitively coupled plasma (CCP), an inductively coupled plasma (ICP), or an arc discharge, more particularly a gliding arc discharge.

In certain embodiments, the plasma is a dielectric barrier discharge or an arc discharge, more particularly a gliding arc discharge.

In particular, the plasma is a non-thermal plasma.

In the scope of the present specification, the term 'non-thermal plasma', designates a plasma which is not in thermal equilibrium, wherein particularly the components of the plasma, such as electrons, ions and neutral particles, comprise distinct temperatures which differ from each other. Non-thermal plasmas are also termed non-equilibrium plasmas.

In particular, the average gas temperature of the plasma in the at least one discharge chamber or the at least one discharge channel is between 10°C and 500°C, particularly between 15° and 250°C, more particularly between 20°C and 150°C. The term 'average gas temperature' relates to the spatial average in the at least one discharge chamber.

In certain embodiments, the device comprises a first electrode and a second electrode, wherein the first electrode and the second electrode are adapted to generate an electric field in the at least one discharge chamber or the at least one discharge channel. In particular, the first and the second electrode extend along the first plane and the second plane.

In particular, the first and the second electrode extend in a plane, which is perpendicular to the first longitudinal axis.

In certain embodiments, the first electrode and the second electrode are positioned outside of the at least one discharge chamber or the at least one discharge channel, wherein a dielectric material is positioned between the first electrode and the at least one discharge chamber or the at least one discharge channel, and between the second electrode and the at least one discharge chamber or the at least one discharge channel, wherein particularly the plasma is a dielectric barrier discharge.

In certain embodiments, the first electrode and the second electrode are positioned in the at least one discharge chamber or the at least one discharge channel, wherein particularly the plasma is an arc plasma, more particularly a gliding arc plasma.

In certain embodiments, a product gas flow is generated in the at least one discharge chamber or the at least one discharge channel or in walls delimiting the at least one discharge chamber or the at least one discharge channel, wherein the product gas flow comprises reactive species, particularly at a concentration of above 1 ppm, more particularly 10 ppm, formed by means of the plasma in the at least one discharge chamber or the at least one discharge channel.

In certain embodiments, the product gas is an aerosol or comprises an aerosol.

In the context of the present specification, the term process gas refers to a gas, gas mixture or a mixture of one or several gases and one or several liquids, from which reactive species can be generated by means of a discharge chamber or the at least one discharge channel.

In certain embodiments, the process gas comprises air, particularly dry air or humid air, water vapor, and/ or a noble gas.

In certain embodiments, the process gas comprises water, particularly distilled water, demineralized water, or tap water, or hydrogen peroxide.

In certain embodiments, the process gas comprises a buffered aqueous solution.

In certain embodiments, the process gas comprises an aqueous solution having a pH below 7, particularly below 4.

In certain embodiments, the at least one discharge chamber comprises a maximum height of 5 cm or less, particularly 1 cm or less, more particularly 0,4 cm or less, extending along a surface normal of the first plane and the second plane, particularly along the first longitudinal axis.

In certain embodiments, the at least one nozzle comprises a maximum height of 5 cm or less, particularly 1 cm or less, more particularly 0,4 cm or less, extending along a surface normal of the first plane and the second plane, particularly along the first longitudinal axis.

In certain embodiments, the at least one discharge channel comprises a maximum height of 5 cm or less, particularly 1 cm or less, more particularly 0,4 cm or less, extending along a surface normal of the first plane and the second plane, particularly along the first longitudinal axis.

In particular, this reduces the necessary weight and space taken up by the device.

In addition, the device according to the present invention can be manufactured in a simpler manner and at reduced costs, because all components of the device can be provided in one main body or several main bodies.

In particular, the height extends perpendicular to a third longitudinal axis along which the device extends, wherein the third longitudinal axis is perpendicular to the first longitudinal axis,.

In certain embodiments, the device comprises a first electrode and a second electrode, which at least partially delimit the at least one discharge chamber or the at least one discharge channel.

In certain embodiments, the first and the second electrode at least partially form the walls of the at least one discharge chamber or the at least one discharge channel.

In particular, the first and the second electrode are adapted to generate an electric field in the at least one discharge chamber or the at least one discharge channel for generating the plasma.

In certain embodiments, the first and the second electrode are positioned in the at least one discharge chamber or the at least one discharge channel.

In certain embodiments, the first and the second electrode are formed from an electrically conductive material, particularly a metal.

In contrast to the cylindrical design of plasma generating devices of the prior art, the planar design of the device according to the present invention facilitates incorporation of two electrodes into the device, for example as part of two half shells forming the at least one discharge chamber or discharge channel. This is advantageous for generating certain types of plasmas, such as arc plasmas.

By means of electrodes in the discharge chamber or the at least one discharge channel, an arc plasma may be generated. In contrast to a dielectric barrier discharge, arc plasmas comprise higher gas temperatures, for example in the range of 1000 K to 3000 K. This is advantageous for generation of certain reactive species, particularly nitrogen monoxide.

In particular, if the distance between the electrodes increases along the path of the process gas and/ or product gas, such as in a nozzle or discharge channel, a gliding arc plasma may be generated. This is advantageous, because in a gliding arc plasma, due to the changing position of the discharge on the electrodes, the abrasion of the electrodes is significantly reduced.

Furthermore, positioning the first and second electrode in the nozzle or the discharge channel has the advantage that the ignition of the arc occurs in an environment of low static pressure, decreasing the necessary ignition voltage. In particular, during a malfunction of the device, in which the pressure in the discharge chamber or the at least one discharge channel increases, plasma generation is automatically interrupted. This provides an inherent protection mechanism which particularly protects an operator of the device against discharge.

In certain embodiments, the at least one discharge chamber is positioned in the at least one nozzle. Such a nozzle with integrated discharge chamber is termed discharge channel in the context of the present specification.

In certain embodiments, the first and the second electrode at least partially form the walls of the at least one nozzle or the at least one discharge channel.

In certain embodiments, the first electrode and the second electrode are positioned in respective walls which at least partially confine the at least one discharge chamber or the at least one discharge channel, wherein the at least one nozzle or the at least one discharge channel is at least partially formed by the walls.

In particular, the first electrode and the second electrode are adapted to generate a gliding arc plasma in the at least one discharge chamber or the at least one discharge channel, particularly the at least one discharge channel.

In certain embodiments, the first electrode and/ or the second electrode comprise a planar geometry.

In certain embodiments, the device comprises at least one suction channel, wherein the at least one discharge chamber is connected to the at least one nozzle by means of the at least one suction channel.

In certain embodiments, the at least one suction channel comprises at least one bend, wherein particularly the at least one suction channel forms a serpentine-shaped pattern. In other words, the suction channel is not straight, thereby elongating the distance traveled by the product gas from the at least one discharge chamber to the nozzle.

In particular, this creates a reaction stretch, in which reactive species comprised in the product gas are dissolved in a second liquid provided in the at least one suction channel or in the at least one discharge chamber. Advantageously, an elongated reaction stretch results in an increased amount of reactive species dissolved in the second liquid.

In certain embodiments, the device comprises at least one suction channel, wherein the at least one discharge chamber is connected to the at least one nozzle by means of the at least one suction channel, and wherein the at least one suction channel comprises at least one bend, wherein particularly the at least one suction channel forms a serpentine-shaped pattern.

In certain embodiments, the at least one suction channel comprises a maximum height of 5 cm or less, particularly 1 cm or less, more particularly 0,4 cm or less, along the first longitudinal axis.

In certain embodiments, the at least one suction channel comprises a length between 5 mm and 500 mm. Therein, in case the at least one suction channel comprises at least one bend, the length extends along the curvature of the at least one bend and corresponds to the distance traveled by the gas flow in the at least one suction channel. Therefore, in this case the length does not extend along a straight line.

In certain embodiments, the at least one suction channel extends between a first plane and a second plane along the first longitudinal axis, wherein the first plane and the second plane are parallel.

In certain embodiments, the device comprises a first half shell and a second half shell wherein the first and the second half shell are joined at a connecting surface which is parallel to the first plane and the second plane, wherein the first half shell and/ or the second half shell form at least a part of the at least one discharge chamber, at least a part of the at least one nozzle, and/ or at least a part of the at least one discharge channel when the first half shell and the second half shell are joined and/ or assembled.

In certain embodiments, the first half shell forms at least a part of the at least one discharge chamber and at least a part of the at least one nozzle when the first half shell and the second half shell are joined. In particular, the at least one discharge chamber and the at least one nozzle are formed by recesses in the first half shell and corresponding surfaces, more particularly flat corresponding surfaces of the second half shell.

In certain embodiments, the first half shell forms at least a part of the at least one discharge channel when the first half shell and the second half shell are joined. In particular, the at least one discharge channel is formed by at least one recess in the first half shell and at least one corresponding surface, more particularly at least one flat corresponding surface of the second half shell.

This is advantageous because recesses have to be introduced into only one part (the first half shell), whereas the second half shell can have a flat surface, thereby reducing complexity and production costs of the device.

In certain embodiments, the first half shell and the second half shell form at least a part of the at least one discharge chamber when the first half shell and the second half shell are joined. Therein, the at least one discharge chamber is formed by corresponding aligned recesses in the first and the second half shell. This has the advantage that a larger volume of the at least one discharge chamber can be achieved in spite of the flat geometry of the device.

In certain embodiments, the first half shell and the second half shell form at least a part of the at least one nozzle when the first half shell and the second half shell are joined. Therein, the at least one nozzle is formed by corresponding aligned recesses in the first and the second half shell. This has the advantage that a larger volume of the at least one nozzle can be achieved in spite of the flat geometry of the device.

In certain embodiments, the first half shell and the second half shell form at least a part of the at least one discharge channel when the first half shell and the second half shell are joined. Therein, the at least one discharge channel is formed by corresponding aligned recesses in the first and the second half shell. This has the advantage that a larger volume of the at least one discharge channel can be achieved in spite of the flat geometry of the device.

Assembling the device from a first half shell and a second half shell has the advantage that the structures, particularly recesses or apertures, necessary to form the at least one discharge chamber, the at least one nozzle, and/ or the at least one discharge channel can be manufactured much easier in separate parts than in one single part, especially when the resulting device has a flat, planar design.

In certain embodiments, the first half shell comprises at least one recess, and the second half shell comprises at least one corresponding surface, wherein at least one cavity is formed by the at least one recess and the at least one corresponding surface, and wherein the at least one nozzle and/or the at least one discharge chamber is formed by the at least one cavity.

In certain embodiments, the device comprises at least one suction channel which is connected to the at least one nozzle and the at least one discharge chamber, such that a gas flow can be generated in the at least one suction channel by means of a pressure gas flow provided in the at least one nozzle, thereby generating the negative pressure in the at least one discharge chamber, wherein the at least one suction channel is formed by the joined first and second half shells, particularly by at least one recess in the first half shell and a corresponding surface of the second half shell or by recesses in the first and second half shell.

In certain embodiments, the at least one suction channel comprises at least one first opening and at least one second opening, wherein the at least one first opening is connected to the at least one nozzle and the at least one second opening is connected to the at least one discharge chamber.

In certain embodiments, the first half shell and/ or the second half shell comprise a sealing material, particularly consist of a sealing material, wherein the sealing material is adapted to seal the at least one discharge chamber and/or the at least one suction channel from the exterior, when the contact pressure is applied.

In certain embodiments, the device comprises a sealing part, which is positioned at the connecting surface between the first and second half shell, wherein the sealing part is adapted to seal the at least one discharge chamber and/ or the at least one suction channel when the contact pressure is applied.

In certain embodiments, the at least one suction channel is positioned in a circumferential direction with respect to the first longitudinal axis.

In certain embodiments, the first half shell and/ or the second half shell is formed from at least two separate parts.

For example, the first and the second half shell may be connected at the connecting surface by joining, bonding, or gluing, or by at least one fixing means, such as a screw.

In certain embodiments, the device comprises at least one connecting piece which is arranged or arrangeable between the first half shell and the second half shell at the connecting surface, wherein the first half shell is joined to the second half shell by means of the connecting piece, and wherein the connecting piece forms at least a part of the at least one discharge chamber, at least a part of the at least one nozzle and/ or at least a part of the at least one discharge channel when the first half shell, the connecting piece, and the second half shell are joined.

In certain embodiments, the at least one connecting piece comprises at least one aperture and/ or at least one recess, particularly at least one aperture, wherein at least a part of the at least one discharge chamber, at least a part of the at least one nozzle and/ or at least a part of the at least one discharge channel is formed by the at least one aperture and/ or the at least one recess and at least one corresponding surface of the first and/ or second half shell.

In certain embodiments, the at least one connecting piece comprises at least one aperture wherein at least a part of the at least one discharge chamber and at least a part of the at least one nozzle is formed by the at least one aperture and at least one corresponding surface of the first and/ or second half shell.

In certain embodiments, the at least one connecting piece comprises at least one aperture wherein at least a part of the at least one discharge channel is formed by the at least one aperture and at least one corresponding surface of the first and/ or second half shell.

In certain embodiments, the at least one connecting piece comprises at least one recess, wherein at least a part of the at least one discharge chamber and/ or at least a part of the at least one nozzle is formed by the at least one recess and at least one corresponding surface of the first and/ or second half shell.

By means of a separate connecting piece, the structures forming the at least one discharge chamber and the at least one nozzle can be realized as apertures in the connecting piece. Therefore, use of the connecting piece advantageously lowers production costs because an aperture (a continuous through-hole or opening) in a part is generally easier to accomplish than a recess (for example a pocket) during manufacture, i.e. by laser cutting.

Furthermore, the connecting piece can extend over the whole device (that is over the first and/ or second half shell), or only over a part of the device, for example in the area forming the at least one discharge chamber in order to increase the size of the at least one discharge chamber for generating more reactive species.

In certain embodiments, first half shell and/ or the second half shell comprise/comprises at least one window formed from a heat resistant material. In particular, the at least one window delimits the at least one discharge chamber or the at least one discharge channel. In particular, the window is formed from a heat-resistant, impact resistant and/ or chemically resistant material, wherein more particularly the window is formed from quartz glass or ceramics.

This is advantageous because the material of the device suffers from stress due to heat and chemically reactive species produced due to plasma generation in the discharge chamber.

In certain embodiments, the device comprises at least one fixing element, wherein the at least one fixing element is adapted to provide a contact pressure between the first and second half shell, and wherein the first half shell and the second half shell comprise overlapping through holes, particularly aligned through-holes, wherein the at least one fixing element extends through the through holes.

In certain embodiments, the device comprises at least one fixing element wherein the at least one fixing element is adapted to provide a contact pressure between the first and second half shell, and wherein the first half shell and the second half shell comprise overlapping through holes, particularly aligned through-holes, wherein the at least one fixing element extends through the through holes, wherein the at least one fixing element is designed as a gas inlet for providing a pressure gas to the at least one nozzle and/ or a process gas to the at least one discharge chamber.

In certain embodiments, the at least one fixing element is designed as a gas inlet for providing a pressure gas and/ or a process gas to the at least one discharge channel.

In certain embodiments, the at least one fixing element is adapted to provide a contact pressure between the first and second half shell, wherein the first half shell and the second half shell comprise overlapping through holes, particularly aligned through-holes, wherein the at least one fixing element extends through the through holes.

In certain embodiments, the at least one fixing element is adapted to provide a contact pressure between the first and second half shell and the connecting piece, wherein the first half shell, the second half shell, and the connecting piece comprise overlapping through holes, particularly aligned through-holes, wherein the at least one fixing element extends through the through holes.

In certain embodiments, the at least one discharge chamber and/ or the at least one suction channel is sealed from the exterior by means of the at least one fixing element.

In certain embodiments, the at least one fixing element is adapted to provide a contact pressure along the first longitudinal axis, such that the at least one discharge chamber and/ or the at least one suction channel is sealed from the exterior.

In certain embodiments, the at least one fixing element extends along a second longitudinal axis, wherein the at least one suction channel is positioned in a circumferential direction with respect to the second longitudinal axis around the at least one though-hole.

In certain embodiments, the first and the second electrode are fixed to the device by means of the at least one fixing element.

In certain embodiments, electrical contacts from a voltage source to the first and the second electrode are provided by means of the at least one fixing element.

Advantageously, the first and the second half shell and/ or the connecting piece can be easily fixed by means of a small number of fixing elements arranged in the through-holes. Thereby, pressure can be provided in order to seal the at least one discharge chamber and the at least one nozzle or the at least one discharge channel from the exterior. In particular this eliminates the need for gluing, bonding, or a large number of fixing elements along the rim of the device, which may more particularly interfere with the proper function of the nozzle or discharge channel.

In certain embodiments, the at least one suction channel extends in a circumferential direction with respect to the at least one through-hole.

In certain embodiments, the at least one fixing element is designed as a gas inlet for providing a pressure gas flow to the at least one nozzle and/ or a process gas flow to the at least one discharge chamber.

In certain embodiments, the at least one fixing element is designed as a gas inlet for providing a pressure gas flow and/ or a process gas flow to the at least one discharge channel.

This feature advantageously eliminates the need for separate fixing elements and gas inlets, thereby reducing the number of required parts, the complexity and the costs of the device.

In certain embodiments, the device comprises at least one gas inlet for providing a gas flow, particularly the pressure gas flow and/ or the process gas flow.

In certain embodiments, the at least one gas inlet comprises a gas port, a channel, and an outlet opening, wherein the gas port is connected to the outlet opening by means of the channel, such that a gas flow, particularly a pressure gas flow or a process gas flow entering the at least one gas inlet travels through the channel to the outlet opening. In particular, the gas port is adapted to be connected to a gas conduit, for example a tubing, such that a gas flow provided in the gas conduit can enter the gas port.

In certain embodiments, the at least one gas inlet comprises a second longitudinal axis, wherein the at least one gas inlet is arranged such that the second longitudinal axis is parallel to the first longitudinal axis.

In certain embodiments, the at least one gas inlet has the shape of a cylinder, wherein the cylinder axis of the cylinder is the second longitudinal axis.

In certain embodiments, the gas port is positioned at the front face, particularly the cylinder base, of the at least one gas inlet.

In certain embodiments, the channel extends along the second longitudinal axis.

In certain embodiments, the outlet opening is positioned radially with respect to the second longitudinal axis.

In certain embodiments, the outlet opening is connected to the at least one nozzle or the at least one discharge channel when the at least one gas inlet, such that the pressure gas flow can be provided in the at least one nozzle or the at least one discharge channel by means of the at least one gas inlet.

In certain embodiments, the outlet opening is connected to the at least one discharge chamber, such that the process gas flow can be provided in the at least one discharge chamber by means of the at least one gas inlet.

In certain embodiments, the at least one gas inlet comprises the fixing element, particularly is the fixing element.

In certain embodiments, the at least one gas inlet comprises an external thread, wherein the device comprises an internal thread, which corresponds to the external thread, particularly wherein the at least one gas inlet is adapted to be fixed at the first and/ or second half shell and/ or the connecting piece by means of the corresponding external and internal threads.

In certain embodiments, the at least one through-hole of the first or second half shell and/ or the connecting piece comprises an internal thread which corresponds to the external thread of the at least one gas inlet, such that the at least one gas inlet may be fixed to the first or second half shell and/ or the connecting piece by means of the external thread and the internal thread.

In particular, the at least one gas inlet is adapted to provide a contact pressure perpendicular to the connecting surface by means of the mechanical connection to the first half shell and the second half shell and/ or the connecting piece.

In certain embodiments, the at least one gas inlet is connected to the first and second half shell and/ or the connecting piece by means of a lock nut, wherein the lock nut comprises an internal thread which is compatible with the external thread of the at least one gas inlet, such that the at least one gas inlet may be fixed to the first and second half shell and/ or the connecting piece by means of the lock nut.

In certain embodiments, the device comprises a pressure gas conduit which is connected to the at least one nozzle or the at least one discharge channel, such that the pressure gas flow can be provided to the at least one nozzle or the at least one discharge channel by means of the pressure gas conduit, and wherein the pressure gas conduit is at least partially arranged adjacent to the at least one discharge chamber, such that the at least one discharge chamber is coolable by means of the pressure gas flow flowing through the pressure gas conduit.

Such a cooling system is advantageous, because materials having a relatively low heat resistance can be used for the device. In particular, other surfaces delimiting the at least one discharge chamber which are not cooled may be manufactured from a heat resistant material.

In certain embodiments, the device comprises a first fluid inlet for providing a fluid in the at least one discharge chamber, particularly upstream of the at least one nozzle or the at least one discharge channel.

In certain embodiments, the device comprises a second fluid inlet for providing a fluid in the at least one nozzle or the at least one discharge channel and/ or in the at least one suction channel, such that the fluid provided in the fluid inlet can be mixed with at least a part of the product gas flow generated in the at least one discharge chamber.

In certain embodiments, the device comprises at least one process gas inlet for providing the process gas flow in the at least one discharge chamber, wherein the at least one process gas inlet is arranged such that the at least one discharge chamber can be cooled by the process gas flow entering the at least one discharge chamber or the at least one discharge channel through the at least one process gas inlet, wherein particularly the at least one process gas inlet is positioned at a proximal side of the at least one discharge chamber with respect to the at least one nozzle.

In certain embodiments, the device comprises at least one process gas conduit which is connected to the at least one discharge chamber, particularly by means of the at least one process gas inlet, such that the process gas flow can be provided to the at least one discharge chamber by means of the process gas conduit, and wherein the process gas conduit is at least partially arranged adjacent to the at least one discharge chamber, such that the at least one discharge chamber is coolable by means of the process gas flow flowing through the process gas conduit.

Such a cooling system is advantageous, because materials having a relatively low heat resistance can be used for the device. In particular, other surfaces delimiting the at least one discharge chamber which are not cooled may be manufactured from a heat resistant material.

In certain embodiments, the device comprises at least one first discharge chamber, at least one second discharge chamber and at least one mixing chamber, wherein the at least one first discharge chamber and the at least one second discharge chamber are connected or connectable to the at least one mixing chamber.

In certain embodiments, the device comprises at least one first discharge channel, at least one second discharge channel, and at least one mixing chamber, wherein the at least one first discharge channel and the at least one second discharge channel are connected or connectable to the at least one mixing chamber, wherein particularly the at least one first discharge channel and/ or the at least one second discharge channel is adapted to generate a gliding arc plasma.

In certain embodiments, the device comprises at least one discharge chamber, at least one discharge channel, and at least one mixing chamber, wherein the at least one discharge chamber and the at least one discharge channel are connected or connectable to the at least one mixing chamber, wherein particularly the at least one discharge channel is adapted to generate a gliding arc plasma.

In certain embodiments, the at least one nozzle is adapted to generate the negative pressure in the at least one first discharge chamber and the at least one second discharge chamber by means of the pressure gas flow flowing through the at least one nozzle.

In certain embodiments, the device is adapted to generate a first plasma from a first process gas flow provided in the at least one first discharge chamber at the negative pressure, wherein the device is adapted to provide a second plasma from a second process gas flow provided in the at least one second discharge chamber at the negative pressure.

In certain embodiments, a first product gas is generated by means of the first plasma and a second product gas is generated by means of the second plasma.

In certain embodiments, the at least one mixing chamber is in flow connection with the at least one first discharge chamber and the at least one second discharge chamber, such that a first product gas flow generated by means of the first plasma and a second product gas flow generated by means of the second plasma can be mixed in the at least one mixing chamber.

In particular, the separate discharge chambers or discharge channels are operated at different pressures, wherein more particularly the different pressures are maintained by means of valves.

Advantageously, separate plasma-generated product gases can be produced by means of the separate discharge chambers or discharge channels at optimized conditions. These product gases can then be mixed in the mixing chamber, in particular such that a further product is formed from the product gases by a chemical reaction.

For some applications, it is advantageous to combine a discharge channel with a discharge channel, for example if a gliding arc plasma (produced by means of the discharge channel) is favored for generation of a product gas, such as in case of NO generation.

In certain embodiments, at least one wall of the at least one discharge chamber (400) comprises a structured surface comprising at least one projection and at least one groove having a depth of 10-1000µm.

In certain embodiments, the at least one discharge chamber is at least partially limited by walls formed from a dielectric material, wherein at least one of the walls comprises a structured surface comprising at least one projection and/ or at least one groove having a depth of 10-1000 µm.

This results in an increased generation of charge carriers in the discharge chamber. In turn, this leads to ignition of a plasma at lower voltages compared to a setup comprising an unstructured surface.

A second aspect of the invention relates to a gas inlet for providing a gas flow, particularly the pressure gas flow and/ or the process gas flow to the device.

In certain embodiments, the gas inlet comprises a gas port, a channel, and an outlet opening, wherein the gas port is connected to the outlet opening by means of the channel, such that a gas flow, particularly a pressure gas flow or a process gas flow entering the gas inlet travels through the channel to the outlet opening. In particular, the gas port is adapted to be connected to a gas conduit, for example a tubing, such that a gas flow provided in the gas conduit can enter the gas port.

In certain embodiments, the gas inlet comprises a second longitudinal axis.

In certain embodiments, the gas inlet has the shape of a cylinder, wherein the cylinder axis of the cylinder is the second longitudinal axis.

In certain embodiments, the gas port is positioned at the front face, particularly the cylinder base, of the gas inlet.

In certain embodiments, the channel extends along the second longitudinal axis.

In certain embodiments, the outlet opening is positioned radially with respect to the second longitudinal axis.

In certain embodiments, the outlet opening is connected to the at least one nozzle or the at least one discharge channel, such that the pressure gas flow can be provided in the at least one nozzle or the at least one discharge channel by means of the gas inlet.

In certain embodiments, the outlet opening is connected to the at least one discharge chamber, such that the process gas flow can be provided in the at least one discharge chamber by means of the gas inlet.

In certain embodiments, the gas inlet comprises the fixing element, particularly is the fixing element.

In certain embodiments, the gas inlet comprises an external thread, wherein the device comprises an internal thread, which corresponds to the external thread, particularly wherein the gas inlet is adapted to be fixed at the first and/ or second half shell and/ or the connecting piece by means of the corresponding external and internal threads.

In certain embodiments, the at least one through-hole of the first or second half shell and/ or the connecting piece comprises an internal thread which corresponds to the external thread of the gas inlet, such that the gas inlet may be fixed to the first or second half shell and/ or the connecting piece by means of the external thread and the internal thread.

In particular, the gas inlet is adapted to provide a contact pressure perpendicular to the connecting surface by means of the mechanical connection to the first half shell and the second half shell and/ or the connecting piece.

In certain embodiments, the gas inlet is connected to the first and second half shell and/ or the connecting piece by means of a lock nut, wherein the lock nut comprises an internal thread which is compatible with the external thread of the gas inlet, such that the gas inlet may be fixed to the first and second half shell and/ or the connecting piece by means of the lock nut.

In certain embodiments, the device comprises at least one liquid inlet for providing a liquid to the at least one discharge chamber, the at least one nozzle, the at least one discharge channel, or the at least one suction channel, wherein particularly the device further comprises at least one vaporizer for generating an aerosol from the liquid at the at least one first liquid inlet.

By means of the flat, planar design of the device, such a liquid inlet can be easily manufactured, for example by forming recesses or apertures in a main body or in modular components of the device.

In certain embodiments, the device comprises a first liquid inlet for providing a liquid to the at least one discharge chamber, wherein particularly the device further comprises at least one vaporizer for generating an aerosol from the liquid. By means of such a liquid inlet, a liquid may be added to the process gas in order to influence plasma chemistry of the generated plasma.

In certain embodiments, the at least one vaporizer comprises a liquid reservoir and a nozzle, particularly a Laval or Venturi nozzle, wherein the nozzle is adapted to mix a gas flow provided in the vaporizer with a liquid comprised in the liquid reservoir, thereby vaporizing the liquid, and wherein the vaporizer is adapted to provide the vaporized liquid through the at least one liquid inlet.

Vaporizing the liquid advantageously results in a complete or almost complete evaporation of the liquid, which particularly increases effectivity of reactive species generation. In particular, a complete evaporation of the liquid can be achieved due to the reduced boiling point of the liquid at the reduced pressure, and/ or due to the heating of the gas/ liquid by means of the plasma produced in the discharge chamber or discharge channel.

In certain embodiments, the device comprises at least one second liquid inlet for providing a liquid to the at least one nozzle or the at least one suction channel, wherein particularly the device further comprises a vaporizer for generating an aerosol from the liquid at the at least one second liquid inlet. By means of such a liquid inlet, components of the product gas, particularly reactive species, may be dissolved in the liquid. Advantageously, the amount of water in the resulting solution may be adjusted independently of the humidity in the at least one discharge chamber.

In certain embodiments, the liquid is water, hydrogen peroxide, and/ or a buffered aqueous solution, particularly water.

In certain embodiments, the liquid comprises carbonic acid and/ or carbon dioxide.

In certain embodiments, the liquid is an aqueous solution having a pH below 7, particularly below 4.

In certain embodiments, the device comprises a microfluidic element, particularly a microfluidic valve.

In certain embodiments, the device comprises an apparatus for water treatment, filtering, distillation, and/ or conditioning of components of the process gas, wherein the apparatus is positioned upstream of the at least one discharge chamber.

In certain embodiments, the device comprises a dispenser which is connected to the at least one discharge chamber or the at least one discharge channel, wherein the dispenser is adapted to eject at least a part of a product gas produced by means of the plasma generated in the at least one discharge chamber or the at least one discharge channel.

In certain embodiments, the dispenser is formed by the joined first and second half shell.

In certain embodiments, the dispenser comprises a baffle plate.

In certain embodiments, the device comprises a dispenser which is formed by the joined first and second half shell and/ or the connecting piece, wherein the dispenser is connected to the at least one discharge chamber or the at least one discharge channel, and wherein the dispenser is adapted to eject at least a part of the plasma generated in the at least one discharge chamber or the at least one discharge channel, wherein the dispenser comprises a baffle plate.

In particular, the dispenser comprises the outlets of the device for ejecting the product gas from the device.

By means of the dispenser, the product gas flow can be evenly distributed on surfaces, particularly on irregular surfaces, for example during use in hand disinfection, closed or half-closed disinfection boxes, in dish washers, or in decontamination of rooms, such as in hospitals.

This is especially advantageous in a planar device because the dispenser can be easily incorporated into parts such as the first and second half shell and the connecting piece.

In particular, no separate baffle plate is needed when a structure of the first or second half shell or the connecting piece is used to decrease the size of liquid drops in the product gas flow.

In certain embodiments, the dispenser is a separate part, which is connectable to the device according to the invention such that the product gas flow may be ejected through the dispenser.

In certain embodiments, the device comprises a casing comprising the dispenser and at least one aperture for inserting a hand, wherein the dispenser comprises a plurality of outlets for ejecting the product gas, wherein the outlets are arranged around the circumference of the at least one aperture.

In certain embodiments, the device comprises a casing comprising the dispenser, wherein the casing comprises at least one aperture for inserting a hand, wherein the dispenser comprises a plurality of outlets, which are arranged around the circumference of the at least one aperture, and wherein the dispenser is adapted to eject at least a part of the plasma generated in the at least one discharge chamber or the at least one discharge channel by means of the outlets, particularly in a radial direction with respect to the at least one aperture.

Such a device is well-suited for hand disinfection.

In particular, the casing comprises a voltage source, more particularly high voltage source, for generating the electric field between the first and second electrode, a compressor or pressure tank for providing the pressure gas flow and/ or the process gas flow, a liquid tank for providing a liquid flow, and/ or a device for water treatment, for example a filter or a distillation unit.

In certain embodiments, the device is comprised in a handpiece comprising the dispenser.

Advantageously, by integration into a handpiece, the device can be manually operated, and fits in small spaces.

In certain embodiments, the device is comprised in a handpiece.

In certain embodiments, the handpiece comprises the dispenser.

In certain embodiments, the handpiece comprises the at least one discharge chamber or the at least one discharge channel, the at least one nozzle, and/ or the first and the second electrode.

A third aspect of the invention relates to a method for generating a plasma, particularly by means of a device according to the first aspect of the invention, comprising the steps of:
- providing a process gas in a discharge chamber or a discharge channel extending between parallel first and second planes, wherein particularly the flow direction of the process gas is perpendicular to a surface normal of the first and the second plane,
- generating a plasma from the process gas, wherein particularly a negative pressure is generated in the discharge chamber or the discharge channel, more particularly by means of a pressure gas flowing through a nozzle which is in flow connection with the discharge chamber or a pressure gas flowing through the discharge channel, wherein the plasma is generated at the negative pressure.

A fourth aspect of the invention relates to a method for generating a reactive species, particularly by means of a device according to the first aspect of the invention, comprising the steps of:
- providing a first process gas, particularly comprising dry air, in a first discharge chamber or a first discharge channel extending between parallel first and second planes,
- providing a second process gas, particularly comprising a mix of water and air, in a second discharge chamber or a second discharge channel extending between parallel first and second planes,
- generating a first reactive species, particularly an NO_{X} species, and/ or a first product gas, particularly comprising an NO_{X} species, from the first process gas by means of a first plasma in the first discharge chamber or the first discharge channel,
- generating a second reactive species, particularly hydrogen peroxide (H₂O₂), and/ or a second product gas, particularly comprising hydrogen peroxide (H₂O₂) from the second process gas by means of a second plasma in the second discharge chamber or the second discharge channel,
- mixing the first reactive species and the second reactive species in a mixing chamber, wherein a third reactive species is formed, particularly wherein the third reactive species is peroxynitrite (ONOO⁻).

In the context of the present specification, the term NO_{X} species designates molecules formed from nitrogen and oxygen atoms, particularly having the molecular formula N₄O, N₂O, N₄O₂, NO, N₂O₃, N₄O₆, NO₂, N₂O₄, N₂O₅., HNO₂, HNO₃, or ONOOH.

In particular, the peroxynitrite is formed from a chemical reaction between hydrogen peroxide and nitrite ions (NO₂⁻), wherein the nitrite ions are formed during mixing of the NO_{X} species with water.

In certain embodiments, the process gas flow is mixed with a first liquid, particularly water, prior to generating the plasma in the at least one discharge chamber or discharge channel.

In certain embodiments, an aerosol is generated from the first liquid, wherein the aerosol is provided in the at least one discharge chamber or discharge channel.

In certain embodiments, the product gas flow is mixed with a second liquid, particularly water, such that reactive species formed by means of the plasma are dissolved in the second liquid.

In certain embodiments, carbon dioxide is mixed with the first liquid and/ or the second liquid, particularly in the device.

In certain embodiments, the process gas is air, particularly ambient air.

In certain embodiments, the process gas is humidified air, water vapor, and/or a noble gas.

In certain embodiments, a high voltage signal is applied to the first and/ or second electrode of the device according to the first aspect.

In certain embodiments, the high voltage signal is an AC signal.

In certain embodiments, the high voltage signal is a DC signal.

In certain embodiments, the high voltage signal is a pulsed signal with rise times in the nanosecond range.

In certain embodiments, the high voltage signal is a sine signal.

In certain embodiments, the high voltage signal comprises a repetition frequency in the range of 100 Hz to 10 MHz.

In certain embodiments, the high voltage signal comprises an amplitude in the range of 100 V to 20 kV.

In certain embodiments, the first plasma and the second plasma are generated, particularly simultaneously, in separate discharge chambers or discharge channels.

In certain embodiments, the aqueous solution has a pH lower than 4, particularly lower than 3,5, more particularly between 2 and 3.

A fifth aspect of the invention relates to a use of the device according to the first aspect of the invention for disinfecting, sterilizing or decontaminating a sample.

In particular, the sample can be a solid, a liquid, or a gas.

The device according to the invention is especially advantageous when used for disinfecting, sterilizing or decontaminating a sample using unstable reactive species, since due to the possible small size and light weight of the device, it can be carried along to the site of use, for example as part of a handheld unit, such that the reactive species can be directly produced on site.

Further features and advantages of the invention will be described hereafter with reference to the figures, wherein
- Fig. 1: shows a perspective view of a first embodiment of the device for generating a plasma according to the invention comprising a first and a second half shell;
- Fig. 2: shows an exploded view of components of the device for generating a plasma according to the first embodiment;
- Fig. 3: shows a cross-sectional view of a fixing element/ gas inlet of the device for generating a plasma according to the first embodiment;
- Fig. 4: shows an exploded view of a second embodiment of the device for generating a plasma according to the invention comprising a first and a second half shell and a connecting piece;
- Fig. 5: shows a schematic cross-sectional view of a third embodiment of the device for generating a plasma according to the invention adapted to generate a gliding arc plasma;
- Fig. 6: shows a cross-sectional view of a fourth embodiment of the device for generating a plasma according to the invention adapted to generate a gliding arc plasma;
- Fig. 7: shows a cross-sectional view of a dispenser of a device for generating a plasma according to the invention;
- Fig. 8: shows a cross-sectional view of a fifth embodiment of the device for generating a plasma according to the invention comprising a first liquid inlet and a vaporizer for generating an aerosol in the discharge chamber;
- Fig. 9: shows a cross-sectional view of a sixth embodiment of the device for generating a plasma according to the invention comprising a second liquid inlet for providing a liquid to the nozzle;
- Fig. 10: shows a cross-sectional view of a seventh embodiment of the device for generating a plasma according to the invention comprising a suction channel comprising bends;
- Fig. 11: shows a cross-sectional view of an eighth embodiment of the device for generating a plasma according to the invention comprising an additional process gas conduit for cooling of the discharge chamber;
- Fig. 12: shows a scheme of a chemical reaction of two chemical species generated by means of a device according to the present invention;
- Fig. 13: shows a cross-sectional view of a ninth embodiment of the device for generating a plasma according to the invention comprising a first and a second discharge chamber and a mixing chamber;
- Fig. 14: shows schematic representations of arrangements of discharge chambers and nozzles in devices according to the present invention;
- Fig. 15: shows a cross-section of a part of a discharge chamber of a device according to the present invention comprising a wall with a structured surface;
- Fig. 16: shows a perspective view of a tenth embodiment of the device for generating a plasma according to the invention comprising apertures with openings for providing the product gas flow;
- Fig. 17: shows a perspective view of an eleventh embodiment of the device for generating a plasma according to the invention comprising a handpiece with openings for providing the product gas flow.

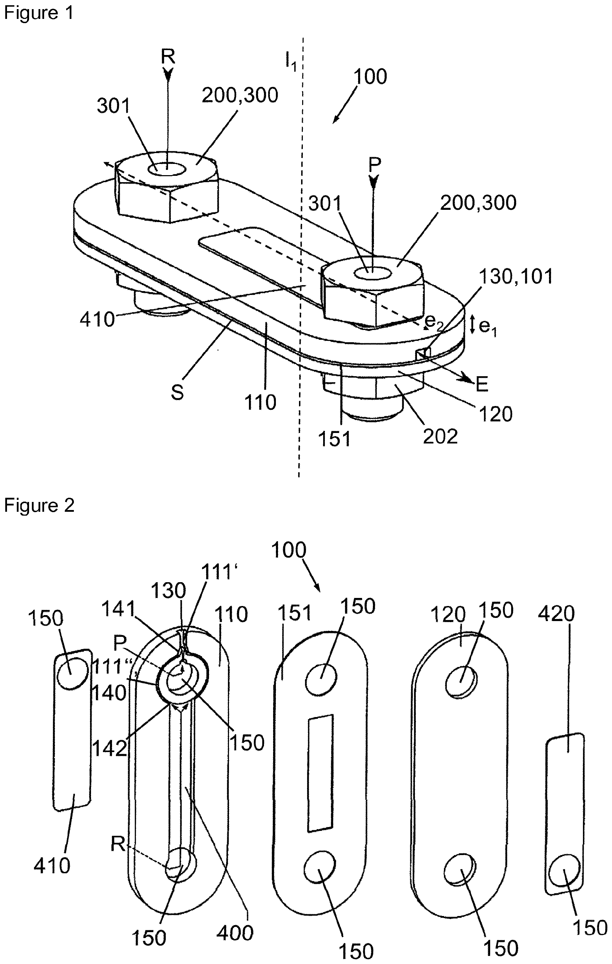

Figure 1 shows a perspective view of the device 100 for generating a plasma comprising a planar first half shell 110, a planar second half shell 120, and a sealing part 151, which are joined at a connecting surface S. The connecting surface S extends perpendicular to a first longitudinal axis l₁.

The first half shell 110 and the second half shell 120 form a discharge chamber 400 (see Fig. 2) and a nozzle 130 which is in flow connection to the discharge chamber 400.

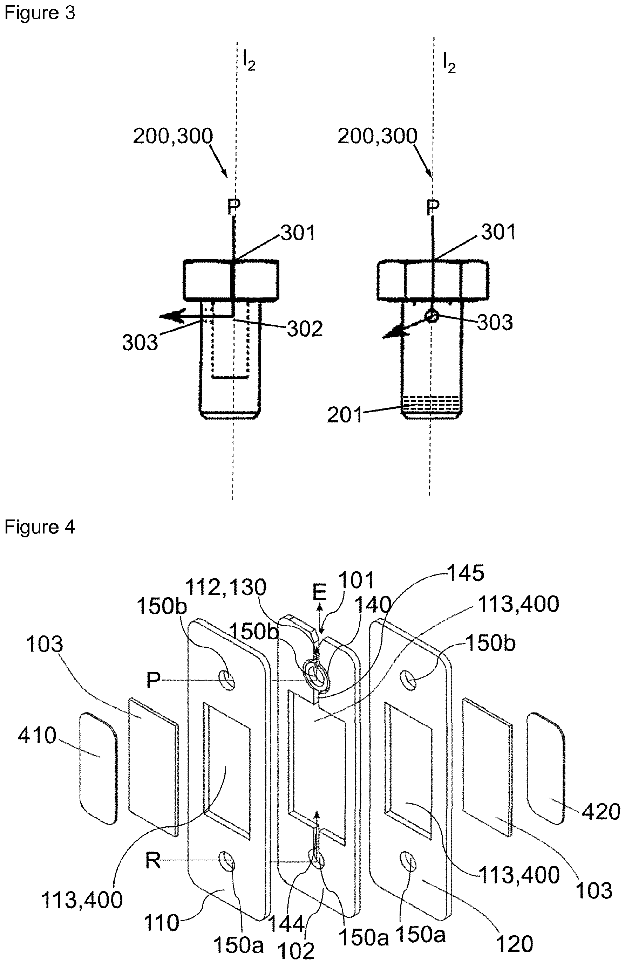

The device 100 further comprises two fixing elements 200 which also serve as gas inlets 300 and extend along a second longitudinal axis l₂ (see Fig. 3) which is arranged parallel to the first longitudinal axis l₁. The gas inlets 300 comprise gas ports 301 for providing a pressure gas flow P to the nozzle 130 and a process gas flow R to the discharge chamber 400. By means of the pressure gas flow P flowing through the nozzle 130, a negative pressure (a partial vacuum) is provided in the discharge chamber 400.

The fixing elements 200 are adapted to exert a mechanical force on the first and second half shell 110,120 which results in a contact pressure between the first and second half shell 110,120. For example, the fixing elements 200 may comprise external threads 201 which correspond to internal threads of the second half shell 120 or a lock nut 202 in order to provide the contact pressure.

Furthermore, Figure 1 shows a planar first electrode 410 which extends perpendicular to the first longitudinal axis l₁, that is in a parallel direction with respect to the connecting surface S. The first electrode 410 is positioned on the first half shell 110, and is mechanically connected to the device 100 by means of one of the combined fixing elements 200/ gas inlets 300.

By means of the first electrode 410 and a second electrode 420 (shown in Figure 2), an electrical field can be provided in the discharge chamber 400, such that a plasma is generated from the process gas flow R in the discharge chamber 400 at the negative pressure.

Figure 1 further shows an outlet 101 of a nozzle 130 (see Fig. 2), wherein the nozzle 130 is formed by the first and second half shell 110,120 (see Fig. 2), and wherein the outlet 101 is formed by a recess 111 (see Fig. 2) of the first half shell 110 and a corresponding surface of the second half shell 120 or the sealing part 151. The outlet 101 is open to the exterior atmosphere and serves to provide a product gas flow E comprising a product gas generated by means of the plasma.

Both the first half shell 110 and the second half shell 120 are characterized by a flat geometry. In particular, the first and the second half shell 110,120 comprise a first maximum extension parallel to the first longitudinal axis l₁ and a second maximum extension e₂ perpendicular to the first longitudinal axis l₁ (depicted in Fig. 1 for the first half shell 110). Therein the second maximum extension e₂ is larger than the first maximum extension e₁ resulting in a flat geometry.

Figure 2 is an exploded view of components of the device 100 depicted in Figure 1, particularly a first electrode 410, a first half shell 110, a sealing part 151, a second half shell 120, and a second electrode 420.

As depicted in Figure 2, the first half shell 110, the second half shell 120, and the sealing part 151 each comprise two respective through holes 150. The through holes 150 are positioned such that corresponding through holes of the first half shell 110, the second half shell 120, and the sealing part 151 overlap, particularly are aligned, when the first half shell 110, the second half shell 120, and the sealing part 151 are assembled to form the device 100.

Therein, the combined fixing elements 200/ gas inlets 300 extend through the overlapping through holes 150, such that a contact pressure can be applied to the first and second half shell 110,120, for example by screwing a lock nut 202 (see Fig. 1) onto an external thread 201 (see Fig. 3) of the respective fixing element 200/ gas inlet 300. Alternatively, the external thread 201 of the fixing elements 200/ gas inlets 300 can be screwed onto corresponding internal threads of the second half shell 120.

The first half shell 110 comprises a first recess 111' extending in a radial direction with respect to the first longitudinal axis l₁ (see Fig. 1). The cross-section of the first recess 111' is narrowest at a central constriction of the first recess 111' and widens in the radial direction (that is toward the outside or circumference of the first half shell 110).

When the two half shells 110,120 are joined at the connecting surface S, the nozzle 130 is formed by the first recess 111' and a flat corresponding surface of the sealing part 151. In absence of a sealing part, the flat corresponding surface can also be formed by the second half shell 120.

The first half shell 110 further comprises a second recess 111" which extends in a circumferential direction with respect to the first longitudinal axis l₁, that is around the central through hole 150. The second recess 111" is connected to the first recess 111' at first openings 141.

When the first and the second half shell 110,120 and the sealing part 151 are joined at the connecting surface S, a suction channel 140 which is connected to the nozzle 130 at first openings 141 and to the discharge chamber 400 at second openings 142 is formed by the second recess 111" of the first half shell 110 and the corresponding surface.

The contact pressure provided by the fixing element 200 (see Fig.1 and Fig. 3) serves to tightly seal the nozzle 130 from the suction channel 140, except at the first openings 141, to ensure an optimal performance of the device 100. In particular, the first half shell 110 and/ or the second half shell 120 may comprise or consist of a sealing material. In this case, an additional sealing part 151 is not necessary and the corresponding surface to form the nozzle 130 and the suction channel 140 is provided by the second half shell 120.

When a pressure gas flow P is provided in the nozzle 130 (i.e. by the gas inlet 300) flowing from the center of the first half shell 110 through the nozzle 130 and exiting the nozzle 130 through the outlet 101, a minimal static pressure is generated in or near the nozzle 130. This minimal static pressure gives rise to a suction gas flow from the discharge chamber 400 through the second openings 142, the suction 140 channel, and the first openings 141 into the nozzle 130, providing the negative pressure in the discharge chamber 400.

If the nozzle 130 is designed as a Venturi nozzle and the pressure gas flow P travels through the nozzle 130 at subsonic speed, the minimal static pressure is generated at the constriction of the nozzle 130. Alternatively, in case the nozzle 130 is designed as a Laval nozzle and the pressure gas flow P travels through the nozzle 130 at supersonic speed, the minimal static pressure is generated downstream of the constriction. In particular, the first openings 141 are arranged at or near the position of the minimal static pressure of the nozzle 130 such that the gas flow through the suction channel 140 is efficiently generated.

Figure 3 shows a combined gas inlet 300/ fixing element 200 in two different views. Figure 3A shows a cross-section and Figure 3B shows a side view of the combined gas inlet 300/ fixing element 200.

The gas inlet 300/ fixing element 200 extends along a second longitudinal axis l₂ and comprises a cylindrical part having a circular cross-section and a hexagonal part with a hexagonal cross-section, i.e. a hexagonal nut. The hexagonal part comprises a gas port 301, and the cylindrical part 310 comprises a channel 302 which is in flow connection with the gas port 301. The cylindrical part 310 further comprises an outlet opening 303 which is arranged in a radial direction with respect to the second longitudinal axis l₂.

Furthermore, the cylindrical part 310 comprises an external thread 201 which is adapted to be connected to a corresponding internal thread, particularly of the second half shell 120 or of an additional lock nut 202 (see Fig. 2).

The gas inlet 300/ fixing element 200 is adapted to provide a contact pressure between the first half shell 110 and the second half shell 120, for example by screwing the external thread 201 into a corresponding internal thread. Therein, as is apparent from Figure 2, the gas inlet 300/ fixing element 200 extends through the aligned through holes 150 of the first and second half shell 110,120.

Furthermore, the gas inlet 300/ fixing element 200 is positioned in the assembled device 100, such that the first longitudinal axis l₁ and the second longitudinal axis l₂ are parallel. Thus, the radially arranged outlet opening 303 of the gas inlet 300 is aligned with the nozzle 130, such that the pressure gas flow P provided through the gas inlet 300 may enter the nozzle 130.

Figure 4 shows a second embodiment of the device 100 for generating a plasma according to the invention, wherein the device 100 comprises a first half shell 110, a second half shell 120, a connecting piece 102, and two windows 103. The discharge chamber 400 is formed by aligned rectangular apertures 113 of the first half shell 110, the second half shell 120, and the connecting piece 102, wherein the discharge chamber 400 is delimited by the windows 103 when the windows 103 are placed on the apertures 113 of the first half shell 110 and the second half shell 120.

In particular, the windows 103 are formed from a resistant material, more particularly from a heat-resistant, impact resistant and/ or chemically resistant material. For example, the windows 103 may be formed from quartz glass or ceramics. Windows 103 formed from a resistant material advantageously resist high temperatures and/ or reactive chemicals generated by means of the plasma in the discharge chamber 400.

The first half shell 110, the second half shell 120, and the connecting piece 102 each comprise a first through-hole 150a and a second through-hole 150b, wherein the first and the second through-holes 105a,105b of the first half shell 110, the second half shell 120, and the connecting piece 102 are aligned when the device 100 is assembled.

The first through-hole 150a of the connecting piece 102 is connected to the aperture 113 of the connecting piece 102 forming the discharge chamber 400 by means of a first channel 144, such that the process gas flow R may be provided to the discharge chamber 400, in particular by means of a gas inlet 300 inserted into the first through-hole 150a.

The device 100 further comprises a nozzle 130 which is formed by a notch 112 in the connecting piece 102 and the corresponding surfaces of the first and second half shell 110,120 when the connecting piece 102 is placed between the first half shell 110 and the second half shell 120 in the assembled device 100. This is in contrast to the first embodiment (Fig. 2) where the nozzle 130 is formed from a recess 111 in the first half shell 110 and a corresponding surface of the second half shell 120 when the first and second half shell 110,120 are assembled to form the device 100.

The nozzle 130 is connected to the second through-hole 150b of the connecting piece 102, such that the pressure gas flow P may be provided to the nozzle 130, in particular by means of a gas inlet 300 inserted into the second through-hole 150b. The connecting piece 102 also comprises a circular recess 111 around the second through-hole 150b, wherein the recess 111 and the corresponding surface of the first half shell 110 form the suction channel 140 when the device 100 is assembled as shown in Figure 4. The suction channel 140 is connected to the aperture 113 of the connecting piece 102 forming the discharge chamber 400 by means of a second channel 145, and the suction channel 140 is connected to the nozzle 130 by means of first openings 141, such that a gas flow from the discharge chamber 400 through suction channel 140 and the nozzle 130 may be generated by the pressure gas flow P flowing through the nozzle 130. Similar to the first embodiment, a negative pressure may be provided in the discharge chamber 400 by means of the suction gas flow from the discharge chamber 400 through the suction channel 140 and the nozzle 130.

Figure 4 further shows a first electrode 410 and a second electrode 420 which are placed onto the windows 103 in the assembled device 100.

The second embodiment of the device 100 shown in Figure 4 can also be described as an embodiment in which the first half shell 110 and the second half shell 120 of the first embodiment (Figure 1) are formed by several parts. Therein, the first half shell 110 shown in Figure 2 would be formed by the first half shell 110 shown in Figure 4, the connecting piece 102, and the window 103, and the second half shell 120 would be formed by the second half shell 120 shown in Figure 4 and the window 103.

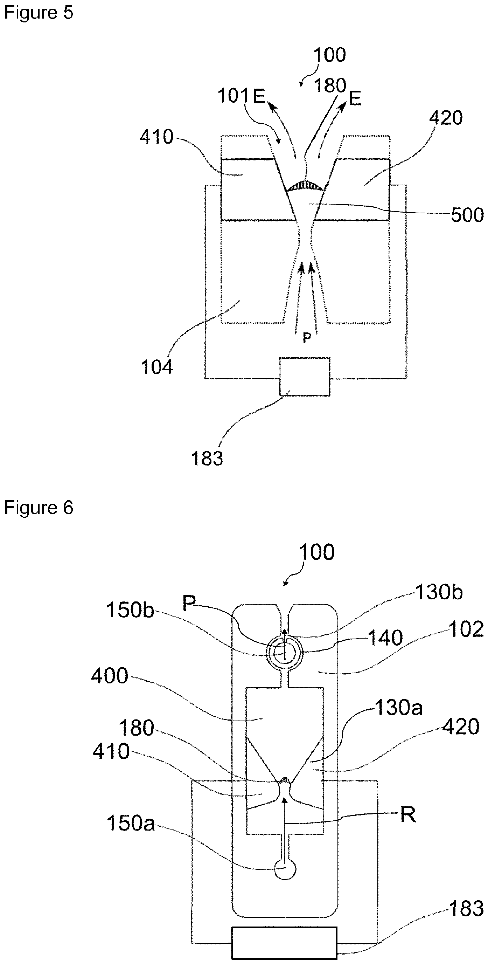

Figure 5 shows a third embodiment of the device 100 for generating a plasma according to the invention comprising a discharge channel 500, which combines the functions of the nozzle 130 and the discharge chamber 400. Therein the first electrode 410 and the second electrode 420, which are particularly formed from an electrically conductive material, more particularly a metal, form parts of walls 104 delimiting the discharge channel 500 of the device 100. In particular, the electrodes 410,420 are planar. In particular, the shape of the electrodes 410,420 is adapted to maintain the shape of the nozzle-shaped discharge channel 500. In particular, the device 100 is adapted such that the front faces of the electrodes 410,420 are in contact with the gas flowing through the discharge channel 500.

The walls 104 of the discharge channel 500 may be completely or partially formed by the electrodes 410,420. In particular, the thickness of the electrodes 410,420 may correspond to the thickness of the walls 104, or the electrodes 410,420 may constitute only the inner part of the walls 104.

The first electrode 410 and the second electrode 420 are electrically connected to a voltage source 183, such that a voltage may be applied between the first electrode 410 and the second electrode 420. The voltage source 183 may be adapted to provide a direct or alternating voltage, which may be continuous or pulsed. The voltage is sufficiently high to produce an arc plasma 180 at the narrowest constriction between the electrodes 410,420.

If the first electrode 420 and the second electrode 420 are formed from a metal, an arc plasma 180 may be generated from a process gas R at the narrowest constriction of the discharge channel 500, which corresponds to the closest distance of the electrodes 410,420, where the electric field between the electrodes 410,420 is strongest. By means of the funnel-like arrangement of the electrodes 410,420 in the discharge channel, the arc plasma 180 may be provided as a gliding arc 180 which periodically glides from the constriction in the direction of the expanding cross-section of the electrodes 410,420 (that is in the direction of the outlet of the discharge channel 500) due to heating of the gas and the resulting lowered particle density.

This has the advantage that the position of the arc is changing over time, such that the abrasion of the electrodes 410,420 is significantly reduced.

The application of a gliding arc plasma 180 is especially advantageous in a planar plasma source, such as the device 100 for generating a plasma according to the present invention, because the electrodes 410,420 can be used as lateral walls of the discharge chamber 400.

Figure 6 shows a cross-sectional view of a fourth embodiment of the device 100 for generating a plasma according to the invention adapted to generate a gliding arc plasma 180. In particular, the depicted part may be a connecting piece 102 analogous to the connecting piece 102 shown in Figure 4, or a first half shell 110 analogous to the first half shell 110 shown in Figure 2.

The depicted device 100 comprises a first electrode 410 and a second electrode comprising an electrically conductive material, , and wherein the first and the second electrode 410,420 form a first nozzle 130a. The first electrode 410 and the second electrode 420 are electrically connected to a voltage source 183, wherein the voltage source 183 is adapted to generate a voltage high enough to produce an arc plasma 180 between the first electrode 410 and the second electrode 420. The discharge chamber 400 of the device 100 is positioned downstream of the first nozzle 130a with respect to a process gas flow R entering the device at the first through-hole 150a, and flowing through the first nozzle 130a.

The device further comprises a second nozzle 130b which is connected to the discharge chamber 400 by means of the suction channel 140.

Advantageously, in the embodiments shown in Figures 5 and 6, since the arc plasma 180 is generated within the nozzle 130 or first nozzle 130a, the static pressure at the position where the arc plasma 180 is generated is reduced, resulting in a reduced minimum voltage to generate the plasma.

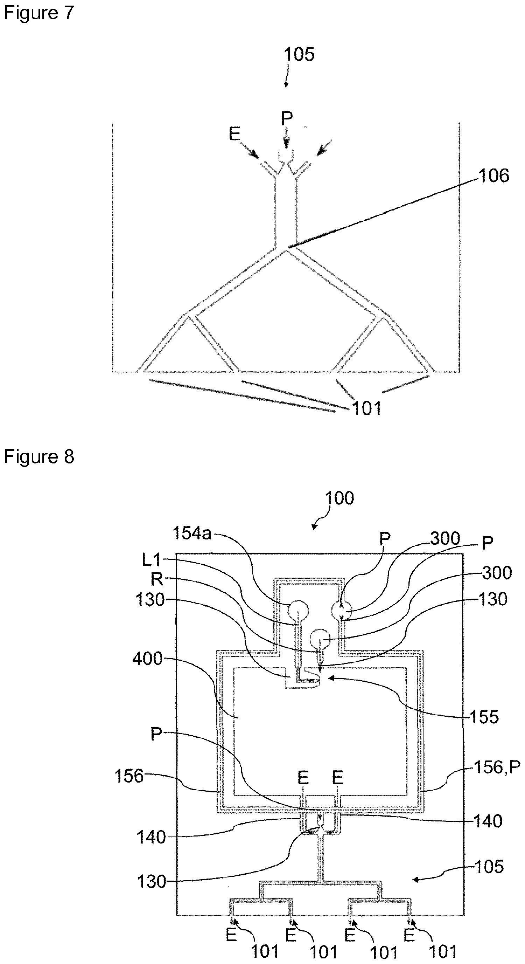

Figure 7 shows a cross-section of a planar dispenser 105 which is part of a device 100 according to the present invention. The dispenser 105 is positioned downstream of the nozzle 130 (or second nozzle 130b) and is adapted to distribute the product gas flow E emerging from the nozzle 130 to a plurality of outlets 101.

In particular, the dispenser 105 is adapted to homogeneously spray the product gas E onto a surface. For example, such a surface may be a body part or a food item to be decontaminated or disinfected by means of the product gas E.

In particular, the gas conducting channels of the dispenser are formed by recesses, for example in the first or second half shell 110,120, or notches/ through-holes in the connecting piece 102. Alternatively, the dispenser 105 may be comprised in a separate part which may be connected to the device 100 according to the invention. In this manner, different dispensers 105 with different arrangements of outlets 101 may be selected for the respective application of the product gas flow E.

In particular, the dispenser 105 comprises a baffle plate 106, which is formed by a part of the first or second half shell 110,120, or the connecting piece 102, wherein the baffle plate 106 is positioned in the flow direction of the product gas flow E, wherein more particularly the baffle plate 106 is adapted to reduce the size of liquid drops mixed with the product gas flow E, most particularly to generate a fine aerosol.

Figure 8 shows a cross-sectional view of a fifth embodiment of the device 100 for generating a plasma according to the invention comprising a first liquid inlet 154a and a vaporizer 155 for generating an aerosol in the discharge chamber 400.

The device 100 comprises a discharge chamber 400, and a gas inlet 300 for providing a process gas flow R into the discharge chamber 400. The device 100 further comprises a nozzle 130, and a gas inlet 300 for providing a pressure gas flow P, wherein the gas inlet 300 is connected to the nozzle 130 by means of a pressure gas conduit 156, and the discharge chamber 400 is connected to the nozzle 130 by means of a suction channel 140.

The pressure gas conduit 156 is arranged adjacent to, particularly around the discharge chamber 400. In particular, the pressure gas conduit 156 is separated from the discharge chamber 400 by a wall which is adapted to conduct heat between the discharge chamber 400 and the pressure gas conduit 156. This has the advantage that the discharge chamber 400 may be cooled by the pressure gas flow P flowing through the pressure gas conduit 156.

Figure 8 further depicts a first liquid inlet 154a for providing a first liquid flow L1 comprising a first liquid into the discharge chamber 400, wherein the first liquid inlet 154a comprises a vaporizer 155, which is adapted to generate an aerosol of the first liquid.

By means of the first liquid inlet 154a a liquid or liquid soluble component may be provided in the discharge chamber 400, wherein particularly a reactive species is formed from the component by means of the plasma generated in the discharge chamber 400. In particularly, the first liquid may be water. More particularly, H₂O₂ may be generated from the water in the discharge chamber 400.

Providing an aerosol comprising the first liquid facilitates almost complete evaporation of the first liquid in the discharge chamber 400.

In addition, the device 100 shown in Figure 8 comprises a dispenser 105 comprising outlets 101 for homogeneously distributing the product gas flow emerging from the nozzle 130.

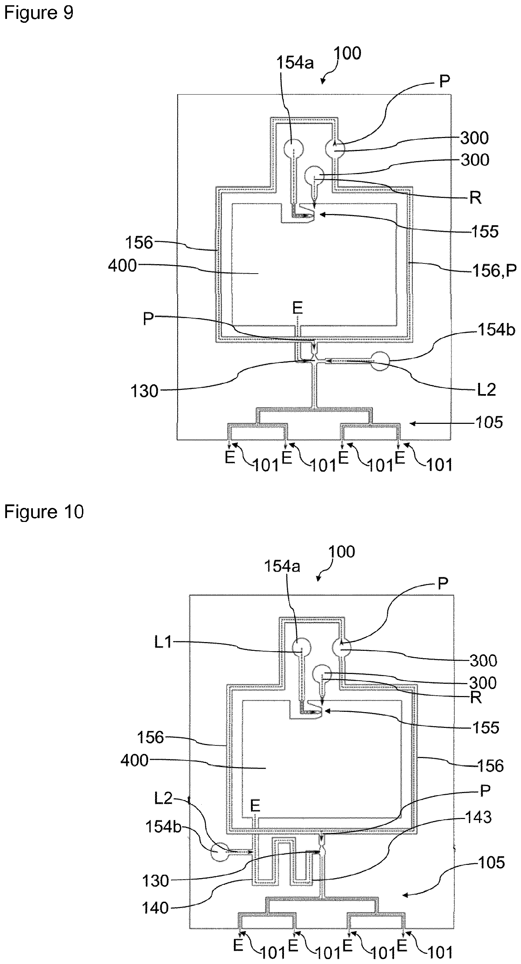

Figure 9 shows a cross-sectional view of a sixth embodiment of the device 100 for generating a plasma according to the invention, wherein the device 100 is designed analogously to the device 100 shown in Figure 8, wherein the device 100 additionally comprises a second liquid inlet 154b for providing a second liquid flow L2 comprising a second liquid to the nozzle 130.

The second liquid inlet 154b is positioned directly at the nozzle 130, such that the pressure gas flow P, the product gas flow E, and the second liquid flow L2 are mixed within the nozzle 130. In particular, the second liquid is provided in order to dissolve a reactive species generated by means of the plasma in the discharge chamber 400.

In particular, the total liquid volume of the first liquid and the second liquid can be adjusted independently from the humidity in the discharge chamber 400. For example, both the first liquid and the second liquid may be water, and the amount of water added to the discharge chamber 400 by means of the first liquid inlet 154a may be chosen to achieve a favorable humidity in the discharge chamber 400, wherein additional water is added to the nozzle 130 by means of the second liquid inlet 154b in order to dissolve reactive species.

The ability to freely adjust the humidity in the discharge chamber 400 is advantageous because changing the humidity changes the occurring plasma chemical processes in the discharge chamber 400.

Figure 10 shows a cross-sectional view of a seventh embodiment of the device 100 for generating a plasma according to the invention, wherein the device 100 is designed analogously to the device 100 shown in Figure 8, wherein the suction channel 140, which connects the discharge chamber 400 to the nozzle 130, additionally comprises bends 143. In other words, the suction channel 140 is not straight, thereby elongating the distance traveled by the product gas flow from the discharge chamber 400 to the nozzle 130. For example, the suction channel 140 may have a serpentine-like shape.

In particular, this creates a reaction stretch, in which reactive species comprised in the product gas flow E are dissolved in the second liquid. Advantageously, an elongated reaction stretch results in an increased amount of reactive species dissolved in the second liquid.

According to the seventh embodiment, the device 100 further comprises a second liquid inlet 154b for providing a second liquid flow L2, which is connected to the suction channel 140 close to the end of the suction channel 140 which is connected to the discharge chamber 400. Thus, the second liquid flow L2 is mixed with the product gas flow E at the beginning of the suction channel 140.

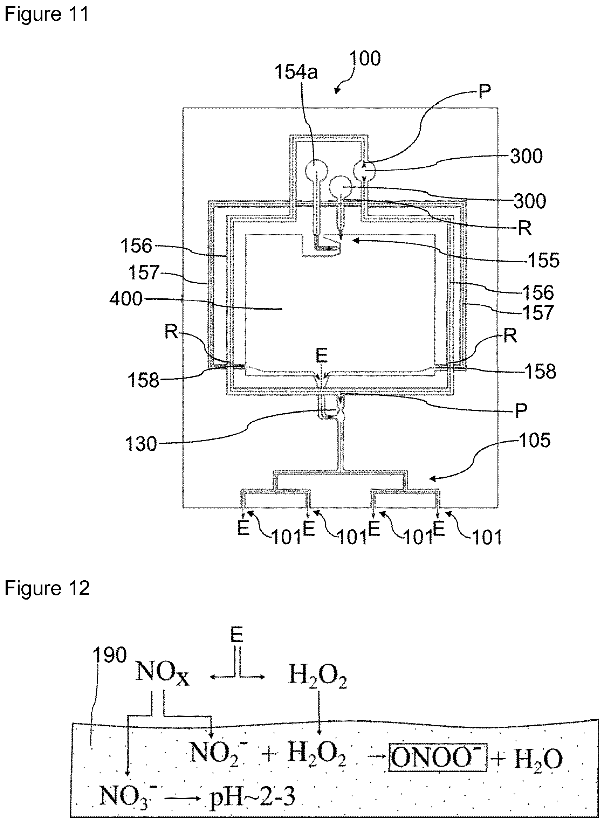

Figure 11 shows a cross-sectional view of a eighth embodiment of the device 100 for generating a plasma according to the invention, wherein the device 100 is designed analogously to the device 100 shown in Figure 8, wherein the device 100 comprises an additional process gas conduit 157 which is connected to the gas inlet 300 providing the process gas flow R. The process gas conduit 157 is positioned adjacent to, particularly around, the discharge chamber 400, and is connected to the discharge chamber 400 by process gas inlets 158 positioned at the side of the discharge chamber 400 which is connected to the nozzle 130.

In particular, the process gas conduit 157 is separated from the discharge chamber 400 by a wall which is adapted to conduct heat between the discharge chamber 400 and the process gas conduit 157. This has the advantage that the discharge chamber 400 may be cooled by the process gas flow R flowing through the process gas conduit 157. In addition, the process gas provided to the discharge chamber 400 by means of the product gas inlets 158 may serve to cool the inside of the discharge chamber 400.

Cooling the discharge chamber 400 at the side which is proximal to the nozzle 130 is advantageous, because the temperature of the gas flow through the discharge chamber 400 increases in flow direction due to continuous plasma generation, and therefore the discharge chamber 400 is exposed to the greatest thermal stress at this location.

Figure 12 shows a scheme of a chemical reaction of NO_{X} and H₂O₂ generated by means of a device 100 according to the present invention. Plasma produced by the device 100 according to the present invention may generate NO_{X} species and/ or H₂O₂ depending on the conditions in the discharge chamber 400.

When the generated NO_{X} species are dissolved in water, nitrite ions (NO₂⁻) as well as nitrate ions (NO₃⁻) are formed. If the resulting aqueous solution also contains dissolved hydrogen peroxide (H₂O₂), a chemical reaction between the nitrite ions and the hydrogen peroxide occurs, resulting in the formation of peroxynitrite ions (ONOO⁻) and water.

In addition, the nitrate ions generated from the NO_{X} species result in a pH of the aqueous solution in the range of 2 to 3. A low pH facilitates the chemical reaction of nitrite ions and hydrogen peroxide to form peroxynitrite ions.

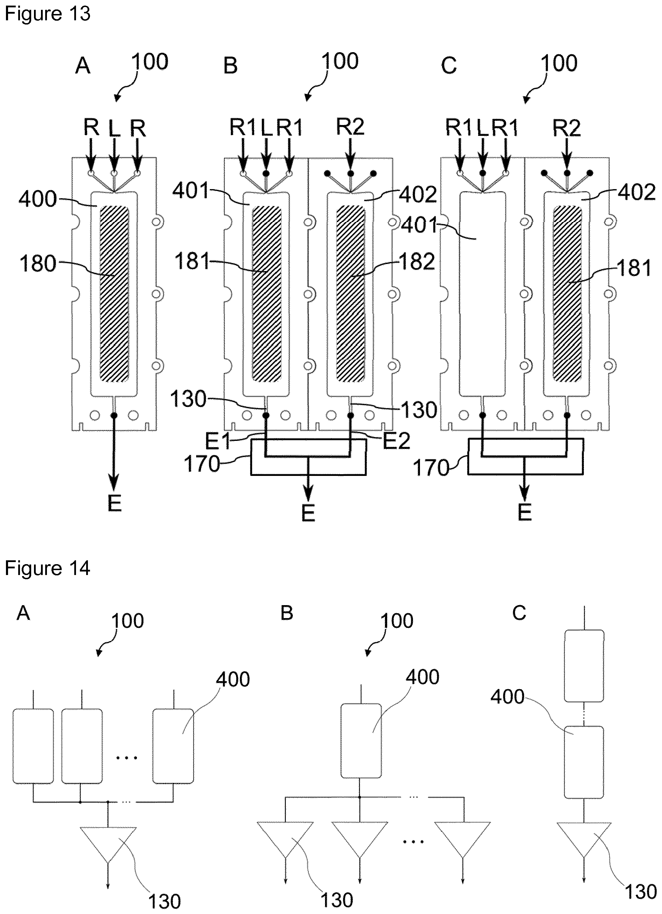

Figure 13 shows a cross-sectional view of different embodiments of the device 100 according to the present invention. Figure 13B and C show a ninth embodiment of the device 100 comprising a first discharge chamber 401 and a second discharge chamber 402 which lead to a mixing chamber 170. Figure 13 A shows an embodiment of the device 100 with only one discharge chamber 400.

Figure 13A shows the generation of a plasma 180 from a process gas R and a liquid L in the discharge chamber 400.

Figure 13B shows the generation of a first plasma 181 in the first discharge chamber 401 and a second plasma 182 in the second discharge chamber 402. In particular, this is achieved by providing a first process gas R1 and a liquid L to the first discharge chamber 401 and a second process gas R2 to the second discharge chamber 402. The first product gas E1 resulting from the first plasma 181 and the second product gas E2 resulting from the second plasma 182 are mixed in the mixing chamber 170, in which a chemical reaction between reactive species generated by the first and the second plasma 181,182 may occur.

Figure 13B shows the generation of the second plasma 182 in the second discharge chamber 402 from the second process gas R2.

In particular, the device 100 shown in Figure 13B and C comprises a first gas inlet 300 for providing the first process gas R1 in the first discharge chamber 401 and a second gas inlet 300 for providing the second process gas R2 in the second discharge chamber 402. In addition, the device 100 may comprise separate liquid inlets for the first and second discharge chamber 401,402.

In particular, the first and the second process gas R1,R2 may be air, and the liquid L may be water. In this manner, given suitable conditions of the first and second plasma 181,182, NO_{X} species may be generated by the first plasma 181, and hydrogen peroxide may be generated by the second plasma 182 in the second discharge chamber 402, wherein nitrite ions are generated from the NO_{X} species in aqueous solution, and wherein particularly peroxynitrite ions are generated from a chemical reaction between the nitrite ions and the hydrogen peroxide in the mixing chamber 170.

Figure 14 shows schematic representations of arrangements of discharge chambers 400 and nozzles 130 in devices 100 according to the present invention, wherein the arrows indicate the direction of process gas flow/ product gas flow. Figure 14A shows an arrangement in which a plurality of discharge chambers 400 are connected to a single nozzle 130, wherein the discharge chambers 400 are connected in parallel. This has the advantage that several reactive species may be generated in parallel in the respective discharge chambers 400, particularly at the respective optimal conditions.

Figure 14B shows an arrangement in which a plurality of nozzles 130 are connected to a single discharge chamber 400, wherein the nozzles 130 are connected in parallel. This has the advantage that a gas flow of a higher flow rate can be generated by the parallel nozzles 130 compared to a single nozzle 130, in particular for providing a high amount of product gas in a short time.

In Figure 14C, an arrangement is depicted in which two discharge chambers 400 are connected in series to a nozzle 130. Alternatively, more than two discharge chambers and/ or more than one nozzle can be connected in series. The parallel and serial connections of discharge chambers 400 and nozzles 130 shown in Figures 14B and 14C can also be combined resulting in more complex connection setups.

In particular, the number of nozzles 130 may be smaller, greater, or equal to the number of discharge chambers 400. Therefore, for example the plurality of discharge chambers 400 shown in Figure 14A may also be connected to more than one nozzle 130, and the plurality of nozzles 130 depicted in Figure 14B may be connected to more than one discharge chamber 400. The device 100 may also comprise two or more discharge chambers 400, wherein each discharge chamber 400 is separately connected to one or several nozzles 130.

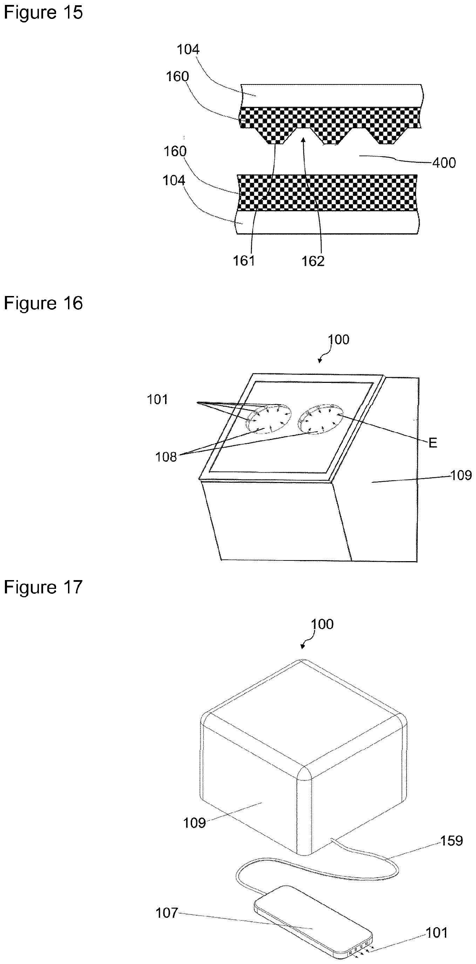

Figure 15 shows a cross-section of a part of the discharge chamber 400 of the device 100 according to the present invention comprising a wall 104, particularly comprising the first electrode 410, with a structured surface 160. The surface 160 comprises alternating projections 161 and grooves 162, particularly forming a regular structure. In particular, the surface 160 of the opposing wall 104, particularly comprising the second electrode 420, may also comprise a similar structure of projections 161 and grooves 162.

In particular, the wall 104 and/ or the surface are formed by a dielectric material. In this case, the electric field generated by the first and second electrode 410,420 is strongest at the positions between the projections 161 and the surface 160 of the opposite wall 104. This results in an increased generation of charge carriers in the discharge chamber 400. In turn, this leads to ignition of a plasma at lower voltages compared to a setup comprising an unstructured surface.

Advantageously, lower ignition voltages allow a reduction of expenses for cable isolation and electrical control.

Figure 16 shows a perspective view of a tenth embodiment of the device 100 for generating a plasma according to the invention comprising a casing 109 comprising two circular apertures 108 with outlets 101 for providing the product gas flow E, wherein the outlets 101 are positioned around the inner circumference of the apertures 108, such that the resulting product gas flows E are directed towards the center of the apertures 108.

In particular, the apertures 108 are designed such that a hand of an operator may be inserted into the respective aperture 108. When the product gas flow E comprises reactive species with an antimicrobial or disinfecting effect, such as peroxynitrite, the device 100 may serve to disinfect the surface of the hands of the operator.

In particular, the casing 109 comprises a voltage source, more particularly high voltage source, for generating the electric field between the first and second electrode 410,420, a compressor or pressure tank for providing the pressure gas flow P and/ or the process gas flow R, a liquid tank for providing the liquid flow L, and/ or a device for water treatment, for example a filter or a distillation unit.

Figure 17 shows a perspective view of an eleventh embodiment of the device 100 for generating a plasma according to the invention comprising a handpiece 107 with outlets 101 for applying the product gas flow E onto a surface.

Components of the device 100, particularly the discharge chamber 400, the first and second electrode 410,420, the nozzle 130 and the dispenser 105, are incorporated in a handpiece 107. The handpiece 107 comprises outlets 101 of the dispenser 105 for distributing the product gas flow E onto a surface.

The device 100 further comprises a casing 109, particularly comprising a voltage source, more particularly high voltage source, for generating the electric field between the first and second electrode 410,420, a compressor or pressure tank for providing the pressure gas flow P and/ or the process gas flow R, a liquid tank for providing the liquid flow L, and/ or a device for water treatment, for example a filter or a distillation unit.

Furthermore, the device 100 comprises a conduit 159 which is adapted to supply the pressure gas flow P, the process gas flow R, voltage, particularly high voltage, for generating the electric field between the electrodes 410,420, and/ or optionally the liquid flow L to the handpiece 107.

The invention is further described by the following example.

### Example: Generation of peroxynitrite by means of separate discharge chambers

It is known from the literature that peroxynitrite (ONOO⁻) as a plasma-derived product considerably contributes to inactivation of bacteria in liquids (Plasma Sources Sci. Technol. 23,2014,015019).

The major reaction of formation of peroxynitrite is the reaction of nitrite (NO₂-) with hydrogen peroxide (H₂O₂). Therein, the rate of formation of peroxynitrite is dependent on the pH of the solution. A considerable formation of peroxynitrite only occurs at a pH below 4.

In order to generate peroxynitrite by means of a plasma source, hydrogen peroxide and NO_{X} species have to be formed by the plasma. These substances dissolve upon contact with liquid and form nitrite (NO₂⁻) and hydrogen peroxide (H₂O₂), which are important educts for peroxynitrite formation. In addition, nitrate ions (NO₃⁻), which are necessary for acidification of the solution, are formed from the NO_{X} species. A scheme of the reaction is depicted in Figure 12.

By means of the planar plasma device according to the present invention having separate first and second discharge chambers, the formation of the educts NO₂⁻ and H₂O₂ can be optimized in such a way that in the resulting plasma-treated liquid preferably equal concentrations of both educts above 10 mg/l are formed.

By separating the discharge chambers, the conditions of the different plasmas for NL₂⁻ and H₂O₂ generation can be selected individually. Since the formation of NO_{X} species, particularly NO, occurs primarily in a dry discharge, whereas humidity (water) is strictly required in the discharge chamber for hydrogen peroxide formation, a separation of the discharge chambers is provided according to the invention.

In the first discharge chamber, dry air is exclusively provided as a first process gas, and NO_{X} species are primarily formed. The second discharge chamber is operated with a mixture of air and liquid, particularly water. Other possible liquids are for example low or high concentration hydrogen peroxide solutions, or acidified or buffered aqueous solutions. Therein, the liquid can be introduced as a film, as drops, a spray or as steam. In the second discharge chamber, hydrogen peroxide is formed from the provided liquid by means of the plasma. This hydrogen peroxide then enters the liquid phase by a dissolution processes, such that a hydrogen peroxide solution is formed in the second discharge chamber.

In particular, both the first and the second discharge chamber are operated at a negative pressure at 50 to 900 mbar.

After passing through the first discharge chamber, the plasma activated gas is mixed with the plasma activated gas liquid mix from the second discharge chamber. At this point, the dissolution of the NO_{X} species takes place, such that after this point hydrogen peroxide as well as NO₂⁻ and NO₃⁻ are present in the liquid. According to a further embodiment, an additional gas or an additional liquid can be added to the mixture at this point.

Figure 13 illustrates the mode of action of the additional discharge chamber. In the case shown in Fig. 13A, water and air were introduced into a single discharge chamber. As a result, a relatively high hydrogen peroxide concentration of over 25 mg/l, but a low nitrite (NO₂⁻) concentration of 5 mg/l was measured.

When two discharge chambers were used, and provided with different process media (a mixture of air and water R1 and dry air R2) such as in the case shown in Fig. 13B, a high nitrite concentration of 20 mg/l was measured, while maintaining a high hydrogen peroxide concentration of over 25 mg/l in the resulting liquid.

This was not the case when no plasma was ignited in the discharge chamber containing water (Fig. 13C, negative control). In this case, the measured nitrite concentration was also 20 mg/l, but no H₂O₂ was detectable. The measured pH of the solution was low in all three cases (pH 2,7 for the single discharge chamber shown in Fig. 13A, pH 3,2 for the two discharge chambers with optimized plasma conditions shown in Fig. 13B, and pH 3,5 in the negative control shown in Fig. 13C). This is favorable for the formation of peroxynitrite.

**List of reference signs**

| | |
|---|---|
| 100 | Device for generating a plasma |
| 101 | Outlet |
| 102 | Connecting piece |
| 103 | Window |
| 104 | Wall |
| 105 | Dispenser |
| 106 | Baffle plate |
| 107 | Handpiece |
| 108 | Aperture |
| 109 | Casing |
| 110 | First half shell |
| 111 | Recess |
| 112 | Notch |
| 113 | Aperture |
| 120 | Second half shell |
| | |
| 130 | Nozzle |
| 130a | First nozzle |
| 130b | Second nozzle |
| 140 | Suction channel |
| 141 | First opening |
| 142 | Second opening |
| 143 | Bend |
| 144 | First channel |
| 145 | Second channel |
| 150 | Through hole |
| 150a | First through-hole |
| 150b | Second through-hole |
| 151 | Sealing part |
| | |
| | |
| | |
| 154a | First liquid inlet |
| 154b | Second liquid inlet |
| 155 | Vaporizer |
| 156 | Pressure gas conduit |
| 157 | Process gas conduit |
| 158 | Process gas inlet |
| 159 | Conduit |
| 160 | Structured surface |
| 161 | Projection |
| 162 | Groove |
| 170 | Mixing chamber |
| 180 | Plasma |
| 181 | First plasma |
| 182 | Second plasma |
| 183 | Voltage source |
| 190 | Liquid phase |
| 200 | Fixing element |
| 201 | External thread |
| 202 | Lock nut |
| 300 | Gas inlet |
| 301 | Gas port |
| 302 | Channel |
| 303 | Outlet opening |
| 400 | Discharge chamber |
| 401 | First discharge chamber |
| 402 | Second discharge chamber |
| 410 | First electrode |
| 420 | Second electrode |
| 500 | Discharge channel |
| l₁ | Surface normal, first longitudinal axis |
| l₂ | Second longitudinal axis |
| S | Connecting surface |
| P | Pressure gas |
| R | Process gas |
| R1 | First process gas |
| R2 | Second process gas |
| E | Product gas |
| E1 | First product gas |
| E2 | Second product gas |
| L | Liquid |
| L1 | First liquid |
| L2 | Second liquid |
| e₁ | First maximum extension |
| e₂ | Second maximum extension |

## Claims

1. A device (100) for generating a plasma (180) and/ or reactive species comprising
- at least one discharge chamber (400) for generating a plasma (180) and at least one nozzle (130) for generating a negative pressure, particularly between 10 mbar and 900 mbar, in the at least one discharge chamber (400), or
- at least one discharge channel (500), wherein the at least one discharge channel (500) is adapted to generate a plasma (180) and a negative pressure, particularly between 10 mbar and 900 mbar, in the discharge channel (500),
**characterized in that**
the at least one discharge chamber (400) and the at least one nozzle (130) or the at least one discharge channel (500) extend between a first plane and a second plane, wherein the first plane and the second plane are parallel.

2. The device (100) according to claim 1, **characterized in that** the at least one discharge chamber (400) and/ or the at least one nozzle (130) or the at least one discharge channel (500) comprises a maximum height of 5 cm or less, particularly 1 cm or less, more particularly 0,4 cm or less, along a surface normal (l₁) of the first plane and the second plane.

3. The device (100) according to claim 1 or 2, **characterized in that** the device (100) comprises a first electrode (410) and a second electrode (420), which at least partially delimit the at least one discharge chamber (400).

4. The device (100) according to one of the preceding claims, **characterized in that** the device (100) comprises at least one suction channel (140), wherein the at least one discharge chamber (400) is connected to the at least one nozzle (130) by means of the at least one suction channel (140), and wherein the at least one suction channel (140) comprises at least one bend (143), wherein particularly the at least one suction channel (140) forms a serpentine-shaped pattern.

5. The device (100) according to one of the preceding claims, **characterized in that** the device (100) comprises a first half shell (110) and a second half shell (120) wherein the first and the second half shell (110,120) are joined at a connecting surface (S) which is parallel to the first plane and the second plane, wherein the first half shell (110) and/ or the second half shell (120) form at least a part of the at least one discharge chamber (400), at least a part of the at least one nozzle (130), and/ or at least a part of the at least one discharge channel (500) when the first half shell (110) and the second half shell (120) are joined.

6. The device according to claim 5, **characterized in that** the device (100) comprises at least one connecting piece (102) which is arranged or arrangeable between the first half shell (110) and the second half shell (120) at the connecting surface (S), wherein the first half shell (110) is joined to the second half shell (120) by means of the connecting piece (102), and wherein the connecting piece (102) forms at least a part of the at least one discharge chamber (400), at least a part of the at least one nozzle (130), and/ or at least a part of the at least one discharge channel when the first half shell (110), the connecting piece (102), and the second half shell (120) are joined.

7. The device (100) according to claim 5 or 6, **characterized in that** the device (100) comprises at least one fixing element (200) wherein the at least one fixing element (200) is adapted to provide a contact pressure between the first and second half shell (110,120), and wherein the first half shell (110) and the second half shell (120) comprise overlapping through holes (150), particularly aligned through-holes (150), wherein the at least one fixing element (200) extends through the through holes (150), wherein the at least one fixing element (200) is designed as a gas inlet (300) for providing a pressure gas (P) to the at least one nozzle (130) and/ or a process gas (R) to the at least one discharge chamber (400) or the at least one discharge channel (500).

8. The device according to one of the preceding claims, **characterized in that**
- the device (100) comprises at least one first discharge chamber (401), at least one second discharge chamber (402), and at least one mixing chamber (170), wherein the at least one first discharge chamber (401) and the at least one second discharge chamber (402) are connected or connectable to the at least one mixing chamber (170), or
- the device (100) comprises at least one first discharge channel, at least one second discharge channel, and at least one mixing chamber (170), wherein the at least one first discharge channel and the at least one second discharge channel are connected or connectable to the at least one mixing chamber (170), or
- the device (100) comprises at least one discharge chamber (400), at least one discharge channel (500), and at least one mixing chamber (170), wherein the at least one discharge chamber (400) and the at least one discharge channel (500) are connected or connectable to the at least one mixing chamber (170).

9. The device (100) according to one of the preceding claims, **characterized in that** at least one wall (104) of the at least one discharge chamber (400) or the at least one discharge channel (500) comprises a structured surface (160) comprising at least one projection (161) and at least one groove (162) having a depth of 10-1000 µm.

10. The device (100) according to one of the preceding claims, **characterized in that** the device (100) comprises at least one liquid inlet (154a, 154b) for providing a liquid (L) to the at least one discharge chamber (400), the at least one nozzle (130), the at least one discharge channel (500), or the at least one suction channel (140), wherein particularly the device (100) further comprises at least one vaporizer (155) for generating an aerosol from the liquid (L).

11. The device (100) according to one of the preceding claims, **characterized in that** the device (100) comprises a dispenser (105) which is connected to the at least one discharge chamber (400) or the at least one discharge channel (500), wherein the dispenser (105) is adapted to eject at least a part of a product gas (E) produced by means of the plasma (180) generated in the at least one discharge chamber (400) or the at least one discharge channel (500).

12. The device (100) according to claim 11, **characterized in that**
- the device (100) comprises a casing (109) comprising the dispenser (105) and at least one aperture (108) for inserting a hand, wherein the dispenser (105) comprises a plurality of outlets (101), which are arranged around the circumference of the at least one aperture (108), or
- the device (100) is comprised in a handpiece (107) comprising the dispenser (105).

13. A method for generating a plasma (180), particularly by means of a device (100) according to one of the claims 1 to 12, comprising the steps of:
- providing a process gas (R) in a discharge chamber (400) or a discharge channel (500) extending between parallel first and second planes, wherein particularly the flow direction of the process gas (R) is perpendicular to a surface normal (l₁) of the first and the second plane ,
- generating a plasma (180) from the process gas (R), wherein particularly
- a negative pressure is generated in the discharge chamber (400), more particularly by means of a pressure gas (P) flowing through a nozzle (130) which is in flow connection with the discharge chamber (400) or the discharge channel (500), wherein the plasma (180) is generated at the negative pressure.

14. A method for generating a reactive species, particularly by means of a device according to one of the claims 1 to 12, comprising the steps of:
- providing a first process gas (R1), particularly comprising dry air, in a first discharge chamber (401) or a first discharge channel extending between parallel first and second planes,
- providing a second process gas (R2), particularly comprising a mix of water and air, in a second discharge chamber (402) or a second discharge channel extending between parallel first and second planes,
- generating a first reactive species, particularly an NO_{X} species, from the first process gas (R1) by means of a first plasma (181) in the first discharge chamber (401) or the first discharge channel,
- generating a second reactive species, particularly hydrogen peroxide (H₂O₂), from the second process gas (R2) by means of a second plasma (182) in the second discharge chamber (402) or the second discharge channel,
- mixing the first reactive species and the second reactive species in a mixing chamber (170), wherein a third reactive species is formed, particularly wherein the third reactive species is peroxynitrite (ONOO⁻).

15. Use of the device (100) according to one of the claims 1 to 12 for disinfecting, sterilizing or decontaminating a sample.
